# EUROPEAN PATENT APPLICATION

(11) **EP 3 117 793 A1**
(43) Date of publication of application: **18.01.2017**
(21) Application number: 16183302.5
(22) Date of filing: 09.11.2011
(51) Int. Cl.: A61B 90/30

(54) **SURGICAL INSTRUMENT INCLUDING ACCESSORY POWERING FEATURE**

(30) Priority: 10.11.2010 US 412140 P; 26.10.2011 US 201113281572
(62) Divisional of application: 11188367.4
(71) Applicant: Covidien LP, Mansfield, MA 02048 (US)
(72) Inventor: VIOLA, Frank, Sandy Hook, Connecticut 06482 (US)
(74) Representative: Soames, Candida Jane

(57) **Abstract**

A surgical instrument system (100), comprising: a surgical instrument (110); an accessory (120) removably mounted to the surgical instrument (110); and an energy device (130) coupled to the accessory (120) while the accessory (120) is coupled to the surgical instrument (110) such that the energy device (130) powers the accessory (120) characterized in that: the accessory (120) includes a first contact (124) and the surgical instrument (110) includes a second contact (115), wherein the energy device (130) transmits power to the accessory (120) upon contact between the first contact (124) and the second contact (115).

## Description

### BACKGROUND

This application claims priority from provisional application serial no. 61/412,140, filed November 10, 2010, the entire contents of which are incorporated herein by reference.

### Technical field

This application relates to surgical instruments and more particularly, to energy sources for use with surgical instrument accessories.

### Background of Related Art

A typical surgery employs a plurality of different surgical instruments and accessory devices for use with the various surgical instruments. When attaching accessory devices, e.g., illumination devices or cameras, there is often a need to satisfy the energy needs of the accessory device. While self contained energy sources like batteries are often utilized, they take up valuable space in the accessory device, thereby increasing the size of the accessory which is disadvantageous in minimally invasive surgery. Additionally, such energy sources often have limited energy storage capacity. As such, removal or repositioning of the accessory may be necessary to change a battery or other energy storage device, which, if required during surgery or other medical procedure, can reduce efficiency and increase the time and complexity of the surgery.

### SUMMARY

Accordingly, the present disclosure in one aspect is directed to a surgical instrument system that includes a surgical instrument, an accessory, and an energy device. The surgical instrument includes a housing, a shaft extending from the housing, and a tool assembly operably coupled to the shaft. The accessory is operably couplable to the surgical instrument. The energy device is operably coupled to the accessory when the accessory is operably coupled to the surgical instrument such that the energy device powers the accessory.

An instrument powering device can be provided in some embodiments which is operably coupled to one or more of the housing, the shaft, and the tool assembly. The instrument powering device can be configured and dimensioned to power the surgical instrument. In some embodiments, the instrument powering device is configured and dimensioned to solely power the tool assembly.

The surgical instrument may include one or more contacts. The one or more contacts may be positioned on the shaft. In some embodiments, one or more contacts of the surgical instrument include one or more slip rings electrically coupled to the energy device.

The accessory may include one or more of a camera and an illumination device. The accessory in some embodiments defines a channel therethrough and one or more contacts. The channel in some embodiments is configured and dimensioned to accommodate at least a portion of the shaft when the accessory is operably coupled to the surgical instrument. The one or more contacts may be positioned within the channel such that when the accessory is positioned on the shaft, where one or more contacts are positioned on the shaft, two or more contacts are engaged so that the accessory is electrically coupled to the energy device.

In some embodiments, the energy device is operably coupled to one or more of the contacts of the accessory, and the energy device transmits power to the accessory when the one or more contacts of the accessory and the one or more contacts of the surgical instrument are in contact. In some embodiments, the energy device includes an energy storage device that is removably positionable within the surgical instrument. The energy storage device may be positionable within the housing of the surgical instrument. In some embodiments, the energy storage device is a battery.

In another aspect, the present disclosure provides a surgical instrument system comprising a surgical instrument having a housing, a shaft extending from the housing, a tool assembly operably coupled to the shaft, and an instrument powering device operably coupled to at least one of the housing, the shaft, and the tool assembly. The instrument powering device is configured and dimensioned to solely power the surgical instrument. An accessory is operably couplable to the surgical instrument, and operably coupled to the instrument powering device when the accessory is operably coupled to the surgical instrument such that the instrument powering device powers the accessory.

In some embodiments, the accessory includes at least one contact and the instrument includes at least one contact, and the at least one contact of the accessory and the at least one contact of the surgical instrument are in contact when the accessory is coupled to the instrument. In some embodiments, the at least one contact of the surgical instrument is positioned on the shaft of the surgical instrument.

In another aspect, the present disclosure provides an accessory for use with a surgical instrument. The accessory is adapted and dimensioned to perform an operation different from, or supplemental to, the operations performed by the surgical instrument. The surgical instrument has a first energy source and at least one electrical contact. The first energy source provides power to the accessory. The accessory includes at least one electrical contact adapted and dimensioned to engage the at least one electrical contact of the surgical instrument in order to electrically couple the accessory to the first energy source.

A second energy source can be provided to power the surgical instrument. In some embodiments, the second energy source provides sole power to the surgical instrument. In some embodiments, the first energy source provides power to solely power the accessory.

### BRIEF DESCRIPTION OF THE DRAWINGS

Various embodiments of the present disclosure are described herein with reference to the drawings wherein:
Fig. 1 is a perspective view of one embodiment of a surgical instrument system in accordance with the present disclosure;
Fig. 1A is a perspective view of another embodiment of the surgical instrument system; and
Fig. 2 is a partial perspective view, with parts separated, of a distal portion of the surgical instrument system of Fig. 1.

### DETAILED DESCRIPTION

Embodiments of the presently disclosed surgical instrument are described in detail with reference to the drawings, wherein like reference numerals designate similar or identical elements in each of the several views. In the drawings and the description that follows, the term "proximal" refers to the end of the surgical instrument that is closer to the user, whereas the term "distal" refers to the end of the surgical instrument that is further from the user.

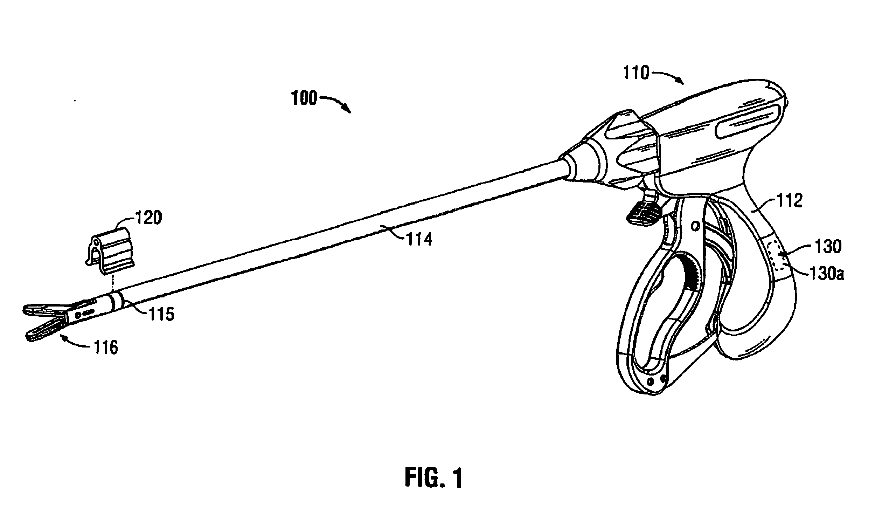

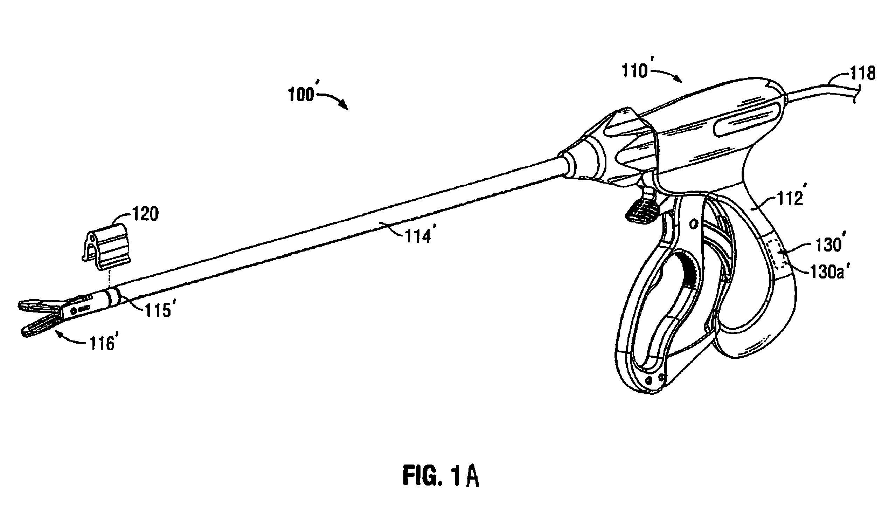

Referring now to the drawings, Fig. 1 illustrates one embodiment of a surgical instrument system 100. The surgical instrument illustrated is a surgical grasper, it being understood that other surgical instruments can be utilized such as endoscopic scissors, clip appliers, surgical staplers, etc. The surgical instrument system 100 includes a surgical instrument 110, an accessory 120, and an energy device 130. The accessory 130 can be powered by external corded power supplies or internal power supplies that are wired internally to contacts on the instrument as described below.

The surgical instrument 110 includes a housing 112, a shaft 114 extending distally from the housing 112, and a tool assembly 116 at a distal end portion of the shaft 114.

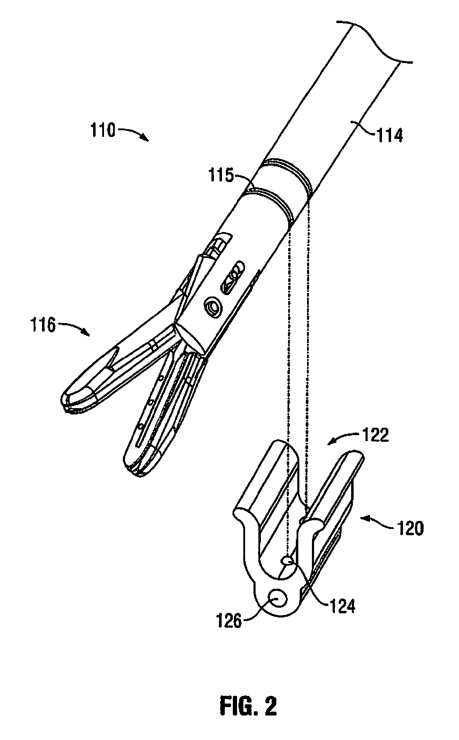

The surgical instrument 110 includes one or more contacts 115. In the illustrated embodiment, two ring contacts 115 are shown by way of example. The contacts 115 may be positioned on the shaft 114. The contacts 115 may include contact buttons, slip rings, or any other suitable connection. The contacts 115 are electrically coupled to the energy storage device 130 (e.g., via one or more wires extending therebetween (through the shaft 114) and/or a wireless connection) such that the energy device 130 transmits power to the contacts 115. The contacts 115 are engaged by the contacts 124 of the accessory 120 (Fig. 2). In this manner, the power is transmitted to the accessory 120 from the energy device 130 through the contacts 115, 124.

Referring now to Fig. 2, the accessory 120 is mountable, and preferably removably mountable, to the surgical instrument 110. The accessory 120 defines a channel 122 therethrough and includes one or more contacts 124. The channel 122 is configured and dimensioned to accommodate at least a portion of the shaft 114 when the accessory 120 is selectively operably coupled to the surgical instrument 110. Thus, the accessory can frictionally engage the shaft 112. Alternatively, the accessory can be attached to the shaft by a snap fit, interlocking structure, or by other methods. The contacts 124 may be positioned within the channel 122 such that when the accessory 120 is positioned on the shaft 114 adjacent contacts 115, two or more contacts (i.e., at least one contact 115 and at least one contact 124) are engaged so that the accessory 120 is electrically coupled to the energy device 130. In particular, the contacts 124 may be operably coupled to the energy device 130 via any suitable electrical or electromechanical, features. For example, these features, which may include wired or wireless connections, may have inductive components, capacitive components, resistive components, switching components, etc. that facilitate the connection between the accessory 120 and the surgical instrument 110 so that the accessory 120 can be powered by the energy device 130. Furthermore, the contacts 124 may include contact buttons, slip rings, or any other suitable connection components. The accessory 120 may include one or more powered devices 126 including cameras, illumination devices, or any other suitable powered devices that could assist the clinician in performing a medical procedure. Consequently, the accessory device can be more compact, i.e. have a reduced profile, as the energy storage device is part of the instrument and not the accessory.

The energy device 130 is operably or electrically coupled with the accessory 120 when the accessory 120 is operably coupled to the surgical instrument 110 such that the energy device 130 powers the accessory 120. In some embodiments, the energy device 130 solely powers the accessory. The energy device 130 is operably or electrically coupled to one or more of the contacts 124 of the accessory 120. The energy device 130 transmits power to the accessory 120 when the one or more contacts 124 of the accessory 120 and the one or more contacts 115 of the surgical instrument 110 are in contact. The energy device 130 includes an energy storage device 130a that may be a battery, rechargeable battery, or any other suitable self-contained electrical energy source. The energy storage device 130a may be removably positionable within the surgical instrument 110 so it can be removed and recharged or removed and replaced with another energy storage device. As seen in Fig. 1, the energy storage device 130a may be positionable within the housing 112, and more particularly in the handle, of the surgical instrument 110. Positioning in other locations in the instrument are also contemplated, as well as positioning outside the instrument.

Although electrical energy sources for the accessory are disclosed herein, other energy sources are also contemplated such as fluid power sources.

In the alternate embodiment of Fig. 1A, the surgical instrument 100' includes an instrument powering device 118 operably coupled to one or more of the housing 112', the shaft 114', and the tool assembly 116'. The instrument powering device 118 is configured and dimensioned to power the surgical instrument 110'. The instrument powering device 118 may be an electrical connection adapted to engage an outlet and/or a generator or any other suitable power source for the reception and/or passage of electrical or electromechanical energy therethrough. In some embodiments, the instrument powering device 118 is configured and dimensioned to solely power the surgical instrument. In some embodiments, it solely powers the tool assembly 116'.

The instrument 100' includes an energy device 130', similar to energy device 130 of Figure 1, positioned in handle 112'. The energy device 130' can alternatively be positioned in other locations in the instrument. In some embodiments, the powering device 118 supplies power to the surgical instrument (e.g. jaws 116') and the energy storage device supplies power to the accessory. In other embodiments, the powering device powers the surgical instrument and the accessory, while the energy storage device provides backup power to the accessory. The powering device can be the sole power source for the instrument.

Instrument 100' has contacts 115' on shaft 114' extending from housing 110' identical to contacts 115 of Figure 1 to provide electrical contact with accessory 120 of Figure 2.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

The invention may be described by reference to the following numbered paragraphs:-
1. A surgical instrument system, comprising:
   a surgical instrument including:
      a housing;
      a shaft extending from the housing; and
      a tool assembly operably coupled to the shaft;
   an accessory operably couplable to the surgical instrument; and
   an energy device operably coupled to the accessory when the accessory is operably coupled to the surgical instrument such that the energy device powers the accessory.
2. The surgical instrument system according to paragraph 1, wherein the accessory includes at least one contact and the surgical instrument includes at least one contact, and the energy device is operably coupled to at least one of the contacts, wherein the energy device transmits power to the accessory when the at least one contact of the accessory and the at least one contact of the surgical instrument are in contact.
3. The surgical instrument system according to paragraph 2, wherein at least one contact of the surgical instrument includes at least one slip ring, the at least one slip ring being electrically coupled to the energy device.
4. The surgical instrument system according to paragraph 2, wherein the accessory defines a channel therethrough, the channel configured and dimensioned to accommodate at least a portion of the shaft when the accessory is operably coupled to the surgical instrument.
5. The surgical instrument system according to paragraph 4, wherein the at least one contact of the surgical instrument is positioned on the shaft and the at least one contact of the accessory is positioned within the channel such that when the accessory is positioned on the shaft, at least two contacts are engaged so that the accessory is electrically coupled to the energy device.
6. The surgical instrument system according to paragraph 1, wherein the energy device includes an energy storage device removably positionable within the surgical instrument.
7. The surgical instrument system according to paragraph 6, wherein the energy storage device includes a battery.
8. The surgical instrument system according to paragraph 6, wherein the energy storage device is positionable within the housing of the surgical instrument.
9. The surgical instrument system according to paragraph 1, wherein the accessory includes at least one of a camera and an illumination device.
10. The surgical instrument system according to paragraph 1, further comprising an instrument powering device operably coupled to at least one of the housing, the shaft, and the tool assembly.
11. The surgical instrument system according to paragraph 10, wherein the instrument powering device is configured and dimensioned to solely power the surgical instrument.
12. The surgical instrument system according to paragraph 10, wherein the instrument powering device is operably coupled to an external generator.
13. A surgical instrument system, comprising:
   a surgical instrument including:
      a housing;
      a shaft extending from the housing; and
      a tool assembly operably coupled to the shaft; and
   an instrument powering device operably coupled to at least one of the housing, the shaft, and the tool assembly, the instrument powering device being configured and dimensioned to solely power the surgical instrument, and
   an accessory operably couplable to the surgical instrument, the accessory being operably coupled to the instrument powering device when the accessory is operably coupled to the surgical instrument such that the instrument powering device powers the accessory.
14. A surgical instrument system according to paragraph 13, wherein the accessory includes at least one contact and the instrument includes at least one contact, and the at least one contact of the accessory and the at least one contact of the surgical instrument are in contact when the accessory is coupled to the instrument.
15. The surgical instrument system according to paragraph 14, wherein the at least one contact of the surgical instrument is positioned on the shaft of the surgical instrument.
16. An accessory for use with a surgical instrument, the surgical instrument having a first energy source and at least one electrical contact, the first energy source providing power to the accessory, the accessory including at least one electrical contact adapted and dimensioned to engage the at least one electrical contact of the surgical instrument in order to electrically couple the accessory to the first energy source so that the first energy source provides power to the accessory, the accessory being adapted and dimensioned to perform an operation different from, or supplemental to, operations performed by the surgical instrument.
17. The accessory of paragraph 16, wherein the surgical instrument includes a second energy source, the second energy source providing power to the surgical instrument.
18. The accessory of paragraph 16, wherein the first energy source provides the power solely to the accessory.
19. The accessory of paragraph 17, wherein the second energy source provides power solely to the surgical instrument.

## Claims

1. A surgical instrument system (100), comprising:
a surgical instrument (110);
an accessory (120) removably mounted to the surgical instrument (110); and
an energy device (130) coupled to the accessory (120) while the accessory (120) is coupled to the surgical instrument (110) such that the energy device (130) powers the accessory (120) **characterized in that**:
the accessory (120) includes a first contact (124) and the surgical instrument (110) includes a second contact (115), wherein the energy device (130) transmits power to the accessory (120) upon contact between the first contact (124) and the second contact (115).

2. The surgical instrument system (100) according to claim 1, wherein the second contact (115) includes a slip ring that is configured to electrically couple to the energy device (130).

3. The surgical instrument system (100) according to claim 1 or 2, wherein the accessory (120) defines a channel (122) therethrough, and the surgical instrument (110) includes a shaft (114), the channel (122) configured to accommodate at least a portion of the shaft (114) therein to selectively operably couple the accessory (120) to the surgical instrument (110).

4. The surgical instrument system (100) according to claim 3, wherein the second contact (115) is positioned on the shaft (114).

5. The surgical instrument system (100) according to claim 3 or claim 4, wherein the first contact (124) is positioned within the channel (122) such that when the accessory (120) is positioned on the shaft (114), the first and second contacts (124), (115) engage one another to electrically couple the accessory (120) to the energy device (130).

6. The surgical instrument system (100) according to any of claims 1-5, wherein the energy device (130) includes an energy storage device (130a) positioned within the surgical instrument (110).

7. The surgical instrument system (100) according to claim 6, wherein the surgical instrument (110) includes a housing (112), and wherein the energy storage device (130a) is removably positioned within the housing (112).

8. The surgical instrument system (100) according to any of claims 1-7, wherein the accessory (120) includes at least one of a camera or an illumination device.

9. The surgical instrument system (100) according to any of claims 1-8, further comprising an instrument powering device (118) coupled to the surgical instrument (110) to solely power the surgical instrument (110).

10. The surgical instrument system (100) according to any of claims 1-8, further comprising an instrument powering device(118) coupled to the surgical instrument (110) to power at least one of the surgical instrument (110) or the accessory (120).

11. The surgical instrument system (100) according to claim 9 or claim 10, wherein the instrument powering device (118) is coupled to a generator.

12. The surgical instrument system (100) according to any of claims 1-11, wherein the accessory (120) is configured to perform an operation different from, or supplemental to, one or more operations performed by the surgical instrument (110).

13. The surgical instrument system (100) according to any of claims 1-12, wherein the accessory (120) is configured to snap-fit connect to the surgical instrument (110).

14. The surgical instrument system (100) according to any of claims 1-12, wherein the accessory (120) includes a plurality of first contacts (124) and the surgical instrument (110) includes a plurality of second contacts (115), the pluralities of first and second contacts (124), (115) configured to contact one another to establish electrical connection between the surgical instrument (110) and the accessory (120).

15. The surgical instrument system (100) according to claim 1, wherein the surgical instrument (110) further comprises a housing (112), a shaft (114) that extends distally from the housing (112) to a distal end portion, and a tool assembly (116) coupled to the distal end portion of the shaft (114).
